(19) Europäisches Patentamt
European Patent Office
Office européen des brevets

(11) Veröffentlichungsnummer: **0 203 220 A1**

# (12) EUROPÄISCHE PATENTANMELDUNG

(21) Anmeldenummer: **85110829.0**

(22) Anmeldetag: **19.08.85**

(51) Int. Cl.4: **C 07 D 417/12**, A 01 N 43/72

(30) Priorität: **17.05.85 DE 3517845**

(43) Veröffentlichungstag der Anmeldung: **03.12.86 Patentblatt 86/49**

(84) Benannte Vertragsstaaten: **CH DE FR GB IT LI**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhansstrasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R., Dr., Im Waldwinkel 110, D-5060 Bergisch Gladbach 2 (DE)**

## (54) Benzolactamsultame.

(57) Die Erfindung betrifft neue Benzolactamsultame der allgemeinen Formel (I)

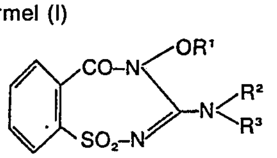

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für den Rest 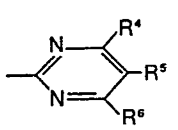

steht, worin

$R^4$ für Wasserstoff, Hydroxy, Halogen oder einen gegebenenfalls substituierten Rest aus der Reihe Alkyl, Alkoxy und Alkylthio steht,

$R^5$ für Wasserstoff, Halogen, Cyano, Formyl oder einen gegebenenfalls substituierten Rest aus der Reihe Alkyl, Alkylcarbonyl und Alkoxycarbonyl steht und

$R^6$ für Wasserstoff, Amino oder einen gegebenenfalls substituierten Rest aus der Reihe Alkyl, Alkoxy, Alkylamino und Dialkylamino steht

– mit der Maßgabe, daß $R^4$ und $R^6$ nicht gleichzeitig für Methyl stehen –,

ein Verfahren zu ihrer Herstellung und ihre Verwendung als Herbizide.

EP 0 203 220 A1

0203220

BAYER AKTIENGESELLSCHAFT 5090 Leverkusen, Bayerwerk

Konzernverwaltung RP Bi/mö
Patentabteilung IVb

Benzolactamsultame

Die Erfindung betrifft neue Benzolactamsultame, ein Verfahren zu ihrer Herstellung und ihre Verwendung als Herbizide.

Benzolactamsultame sind bisher nicht aus der Literatur bekannt. Verschiedene Benzolactamsultame sind Gegenstand von nicht zum vorveröffentlichten Stand der Technik gehörenden Patentanmeldungen der Anmelderin (vergl. DE-OS 3 431 915, 3 431 920 und 3 431 927).

Es wurden nun neue Benzolactamsultame der allgemeinen Formel (I)

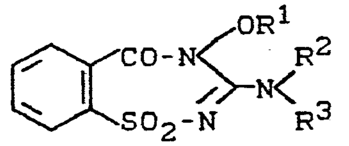 (I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für den Rest 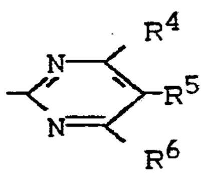 steht, worin

$R^4$ für Wasserstoff, Hydroxy, Halogen oder einen gegebenenfalls substituierten Rest aus der Reihe Alkyl, Alkoxy und Alkylthio steht,

$R^5$ für Wasserstoff, Halogen, Cyano, Formyl oder einen gegebenenfalls substituierten Rest aus der Reihe Alkyl, Alkylcarbonyl und Alkoxycarbonyl steht und

$R^6$ für Wasserstoff, Amino oder einen gegebenenfalls substituierten Rest aus der Reihe Alkyl, Alkoxy, Alkylamino und Dialkylamino steht,

- mit der Maßgabe, daß $R^4$ und $R^6$ nicht gleichzeitig für Methyl stehen -

gefunden.

Man erhält die neuen Verbindungen der Formel (I), wenn man 2-Chlorsulfonyl-benzoylchlorid der Formel (II)

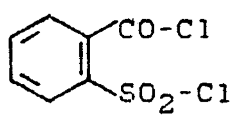

(II)

mit Oxyguanidin-Derivaten der Formel (III)

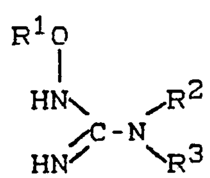

(III)

in welcher

$R^1$, $R^2$ und $R^3$ die oben angegebenen Bedeutungen haben,

in Gegenwart von Säureakzeptoren und gegebenenfalls in Gegenwart von Verdünnungsmitteln umsetzt.

Die neuen Benzolactamsultame der Formel (I) zeichnen sich durch starke herbizide Wirksamkeit aus.

Überraschenderweise zeigen die neuen Verbindungen der Formel (I) erheblich stärkere herbizide Wirkung als viele bekannte chemische Vebindungen gleicher Wirkungsrichtung.

Gegenstand der Erfindung sind vorzugsweise Verbindungen der Formel (I), in welcher

$R^1$ für $C_1$-$C_{12}$-Alkyl [welches gegebnenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkyl-amino-carbonyl oder Di-($C_1$-$C_4$-Alkyl)-aminocarbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$--Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy--carbonyl substituiert ist] oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht, in welcher weiter

$R^2$ für Wasserstoff, $C_1$-$C_4$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl], $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] steht, in welcher ferner

$R^3$ für den Rest 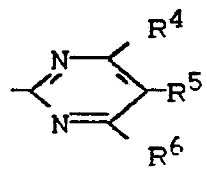 steht, worin

$R^4$ für Wasserstoff, Hydroxy, Fluor, Chlor, Brom, für $C_1$-$C_4$-Alkyl, $C_1$-$C_4$-Alkoxy oder $C_1$-$C_4$-Alkylthio [welche gegebenenfalls durch Fluor und/oder Chlor substituiert sind] steht,

$R^5$ für Wasserstoff, Fluor, Chlor, Brom, Cyano, Formyl, $C_1$-$C_4$-Alkyl [welches gegebenenfalls durch Fluor und/oder Chlor substituiert ist], für $C_1$-$C_4$-Alkyl-carbonyl oder $C_1$-$C_4$-Alkoxy-carbonyl steht und

$R^6$ für Wasserstoff, Amino, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkyl-amino oder Di-($C_1$-$C_4$-alkyl)-amino steht

- mit der Maßgabe, daß $R^4$ und $R^6$ nicht gleichzeitig für Methyl stehen.

Gegenstand der Erfindung sind insbesondere Verbindungen der Formel (I), in welcher

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenylethyl oder Benzyl [welches gegebenenfalls durch Fluor, Chlor, Nitro, Cyano, Methyl, Methoxy oder Methoxy-carbonyl substituiert ist], steht,

$R^2$ für Wasserstoff steht und

$R^3$ für den Rest 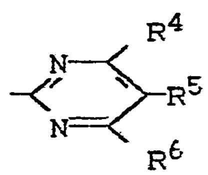 steht, worin

$R^4$ für Wasserstoff, Chlor oder $C_1$-$C_2$-Alkoxy [welches gegebenenfalls durch Fluor und/oder Chlor substituiert ist], steht,

$R^5$ für Wasserstoff, Methylcarbonyl oder $C_1$-$C_2$-Alkoxy-carbonyl steht und

$R^6$ für Wasserstoff, $C_1$-$C_2$-Alkyl [welches gegebenenfalls durch Fluor und/oder Chlor substituiert ist] oder $C_1$-$C_2$-Alkoxy [welches gegebenenfalls durch Fluor und/oder Chlor substituiert ist] steht.

Verwendet man beim erfindungsgemäßen Verfahren beispielsweise N'-(4-Methyl-pyrimidin-2-yl)-N''-allyloxy-guanidin und 2-Chlorsulfonyl-benzoylchlorid als Ausgangsstoffe, so kann der Reaktionsablauf durch folgendes Formelschema skizziert werden:

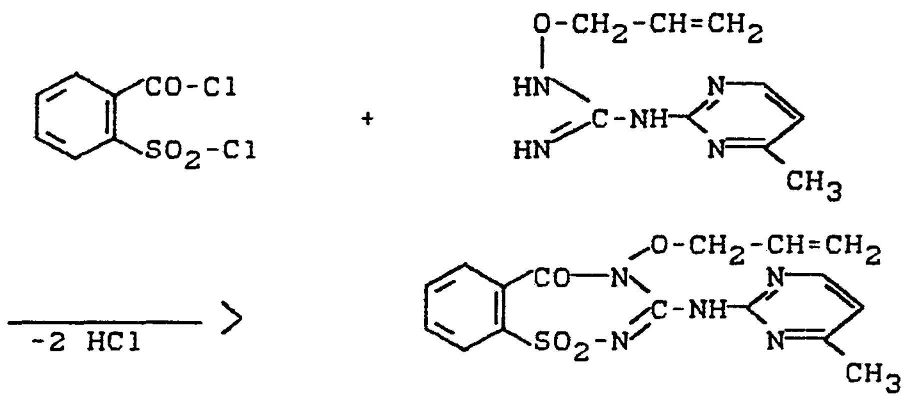


Das beim erfindungsgemäßen Verfahren als Ausgangsstoff zu verwendende 2-Chlorsulfonyl-benzoylchlorid der Formel (II) ist bereits bekannt (vergl. DE-OS 2 036 171).

Die beim erfindungsgemäßen Verfahren weiter als Ausgangsstoffe zu verwendenden Oxyguanidin-Derivate sind durch die Formel (III) allgemein definiert. In Formel (III) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (III) seien beispielsweise genannt:

N'-(4-Methyl-pyrimidin-2-yl)-,
N'-(4-Methoxy-6-methyl-pyrimidin-2-yl)-,

N'-(4-Ethyl-pyrimidin-2-yl)-,
N'-(4-Ethoxy-6-methyl-pyrimidin-2-yl)-,
N'-(4-Chlor-6-methoxy-pyrimidin-2-yl)-,
N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)-, und
N'-(4-Difluormethoxy-6-methyl-pyrimidin-2-yl)-
-N''-methoxy-guanidin
-N''-ethoxy-guanidin,
-N''-propoxy-guanidin,
-N''-isopropoxy-guanidin,
-N''-butoxy-guanidin,
-N''-isobutoxy-guanidin,
-N''-sec,-butoxy-guanidin,
-N''-pentoxy-guanidin,
-N''-isopentoxy-guanidin,
-N''-hexyloxy-guanidin,
-N''-octyloxy-guanidin,
-N''-allyloxy-guanidin,
-N''-(2-chlor-ethoxy)-guanidin,
-N''-(2-fluor-ethoxy)-guanidin,
-N''-(2-chlor-propoxy)-guanidin,
-N''-(2-fluor-propoxy)-guanidin,
-N''-(3-chlor-propoxy)-guanidin,
-N''-(4-chlor-butoxy)-guanidin,
-N''-methoxycarbonylmethoxy-guanidin,
-N''-ethoxycarbonylmethoxy-guanidin,
-N''-(1-methoxy-carbonyl-ethoxy)-guanidin,

-N''-(1-ethoxy-carbonyl-ethoxy)-guanidin,
-N''-(dimethylamino-carbonyl-methoxy)-guanidin,
-N''-(2-phenyl-ethoxy)-guanidin,
-N''-phenoxy-guanidin,
-N''-(4-methyl-benzyloxy)-guanidin,
-N''-(4-fluor-benzyloxy)-guanidin,
-N''-(4-chlor-benzyloxy)-guanidin,
-N''-(4-nitro-benzyloxy)-guanidin,
-N''-(2,6-dichlor-benzyloxy)-guanidin,
-N''-(4-methoxycarbonyl-benzyloxy)-guanidin und
-N''-(4-ethoxycarbonyl-benzyloxy)-guanidin.

Die Ausgangsstoffe der Formel (III) sind zum Teil bekannt (vergl. J. Chem. Soc. 1962, 3915 und EP-A 121 082).

Man erhält die Verbindungen der Formel (III) wenn man Cyanamid-Derivate der Formel (IV)

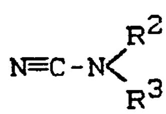
(IV)

in welcher

$R^2$ und $R^3$ die oben angegebenen Bedeutungen haben,

mit Hydroxylamin-Derivaten der Formel (V)

$$H_2N\text{-}OR^1 \qquad (V)$$

in welcher

$R^1$ die oben angegebene Bedeutung hat,

bzw. deren Hydrochloriden,

gegebenenfalls in Gegenwart von Verdünnungsmitteln, wie z. B. Ethanol, Propanol oder Butanol, bei Temperaturen zwischen 20 °C und 120 °C umsetzt und gegebenenfalls die Umsetzungsprodukte mit Säureakzeptoren, wie z. B. Ammoniak, Kaliumcarbonat oder Natriumhydroxid, behandelt.

Die Cyanamid-Derivate der Formel (IV) sind zum Teil bekannt (vergl. J. Chem. Soc. 1953, 1725). Man erhält die Verbindungen der Formel (IV) im wesentlichen nach folgenden Synthesewegen:

(a) durch Umsetzung von Alkalimetall- oder Erdalkalimetall-Salzen von Cyanamid - wie z. B. Natriumcyanamid oder Calciumcyanamid - mit Chlor-hetarenen der Formel (VI)

$$Cl\text{-}R^3 \qquad (VI)$$

in welcher

$R^3$ die oben angegebene Bedeutung hat,

und gegebenenfalls anschließend - wenn $R^2$ nicht für Wasserstoff steht - mit Halogenverbindungen der Formel (VII)

$$Q\text{-}R^2 \qquad (VII)$$

in welcher

$R^2$ für einen gegebenenfalls substituierten Rest aus der Reihe Alkyl, Alkenyl, Alkinyl und Aralkyl steht, und

Q für Chlor, Brom oder Iod steht,

gegebenenfalls in Gegenwart von inerten Verdünnungsmitteln, wie z. B. Aceton, Acetonitril oder Dimethylformamid, bei Temperaturen zwischen 0 °C und 100 °C.

Nach Einengen und Auflösen des Rückstandes in Wasser können die Cyanamid-Derivate der Formel (IV) durch Ansäuern, z. B. mit Salzsäure, ausgefällt und durch Absaugen isoliert werden.

Alternativ erhält man die Verbindungen der Formel (IV)

(b) durch Umsetzung von Cyanoguanidin ('Dicyandiamid') mit β-Dicarbonylverbindungen oder deren Derivaten,

wie z. B. Acetylaceton (vergl. J. Chem. Soc. 1953, 1725 - 1730), Acetessigsäureestern (vergl. J. Prakt. Chem. 77 (1908), 542 und J. Chem. Soc. 1948, 586) oder Malonsäureestern (vergl. DE-PS 158 591).

Die aus Acetessigsäureestern bzw. Malonsäureestern erhaltenen 2-Cyanamino-4-hydroxy-6-methyl- bzw. -4,6-dihydroxy-pyrimidine können auf bekannte Weise durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z. B. Acetanhydrid oder Acetylchlorid acyliert und nach der Alkylierung mit wässrigen Säuren oder Basen wieder entacyliert.

In einem weiteren Alternativverfahren erhält man die Verbindungen der Formel (IV), wenn man

(c) Amino-hetarene der Formel (VIII)

$H_2N-R^3$ (VIII)

in welcher

$R^3$ die oben angegebene Bedeutung hat,

mit Carbonylisothiocyanaten der Formel (IX)

```
   O
   ||
R7-C-N=C=S
```

$R^7-C(=O)-N=C=S$ (IX)

in welcher

$R^7$ für Ethoxy oder Phenyl steht,

gegebenenfalls in Gegenwart eines inerten Verdünnungsmittels, wie z. B. Aceton, Acetonitril oder Toluol, bei Temperaturen zwischen 0 °C und 100 °C umsetzt, die hierbei gebildeten Carbonylthioharnstoffe der Formel (X)

```
   O     S
   ||    ||
R7-C-NH-C-NH-R3
```

(X)

in welcher

$R^3$ und $R^7$ die oben angegebenen Bedeutungen haben,

gegebenenfalls nach Einengen durch Absaugen isoliert und mit wässrigen Alkalimetall- oder Erdalkalimetall-hydroxidlösungen, wie z. B. Natronlauge, gegebenenfalls in Gegenwart eines organischen Lösungsmittels, wie z. B. Tetrahydrofuran oder Dioxan, bei Temperaturen zwischen 0 °C und 120 °C umsetzt und die nach Ansäuern, z. B. mit Salzsäure, kristallin erhaltenen Thioharnstoffe der Formel (XI)

$$H_2N-\overset{\overset{S}{\|}}{C}-NH-R^3 \qquad (XI)$$

in welcher

$R^3$ die oben angegebene Bedeutung hat,

durch Absaugen isoliert und mit Metallverbindungen, welche Schwefelwasserstoff binden können, wie z. B. mit Blei(II)-acetat, Kupfer(II)-acetat, Quecksilber(II)-acetat oder Eisen(II)-acetat, in Gegenwart

von wässrigen Alkalimetall- oder Erdalkalimetall--hydroxidlösungen, wie z. B. Natronlauge, bei Temperaturen zwischen 20 °C und 100 °C umsetzt, nach Ende der Umsetzung filtriert und das Filtrat mit einer Säure, wie z. B. Essigsäure, ansäuert. Die hierbei kristallin anfallenden Produkte der Formel (IV) können durch Absaugen isoliert werden.

Die Ausgangsstoffe für die vorausgehend unter (a), (b) und (c) beschriebenen Herstellungsverfahren für die Cyanamid-Derivate der Formel (IV) sind bekannt und/oder können nach an sich bekannten Verfahren hergestellt werden.

Hierzu gehören die Chlor-hetarene der Formel (VI) (vergl. J. Chem. Soc. (C) 1966, 2031; Chem. Pharm. Bull. 11 (1963), 1382 - 1388 und Arch. Pharm. 295 (1962), 649 - 657), die Halogenverbindungen der Formel (VII) (handelsübliche Chemikalien), die Amino-hetarene der Formel (VIII) (vergl. Chem. Pharm. Bull. 11 (1963), 1382 - 1388; J. Chem. Soc. 1946, 81 und US-PS 4 299 960) und die Carbonylisothiocyanate der Formel (IX) (vergl. J. Heterocycl. Chem. 5 (1968), 837 und US-PS 4 160 037).

Das erfindungsgemäße Verfahren zur Herstellung der neuen Verbindungen der Formel (I) wird vorzugsweise unter Verwendung von Verdünnungsmitteln durchgeführt. Als solche kommen praktisch alle inerten organischen, vorzugsweise

jedoch aprotisch polare Solventien in Betracht. Hierzu gehören gegebenenfalls halogenierte Kohlenwasserstoffe, wie z. B. Methylenchlorid, Chloroform, Toluol und Chlorbenzol, Nitrile, wie z. B. Acetonitril und Propionsäurenitril, Dimethylformamid, Dimethylacetamid, Dimethylsulfoxid, Sulfolan, Hexamethylphosphorsäuretriamid, 1,2--Dimethoxyethan, Pyridin und 2-Methyl-5-ethyl-pyridin.

Als Säureakzeptoren können beim erfindungsgemäßen Verfahren praktisch alle üblicherweise verwendeten Säurebindemittel eingesetzt werden. Hierzu gehören insbesondere Alkalimetall- und Erdalkalimetall-hydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicycloundecen (DBU), Pyridin, 2-Methyl-5-ethyl-pyridin und 4-Dimethylamino-pyridin.

Die Reaktionstemperaturen können beim erfindungsgemäßen Verfahren innerhalb eines größeren Bereichs variiert werden. Im allgemeinen arbeitet man zwischen -80 °C und +100 °C, vorzugsweise zwischen -40 °C und +50 °C. Das erfindungsgemäße Verfahren wird im allgemeinen bei Normaldruck durchgeführt.

Zur Durchführung des erfindungsgemäßen Verfahrens setzt man je Mol Oxyguanidin-Derivat der Formel (III) im allgemeinen zwischen 1,0 und 1,5 Mol, vorzugsweise zwischen

1,0 und 1,2 Mol 2-Chlorsulfonyl-benzoylchlorid der Formel (II) ein. Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen der Formel (I) kann nach üblichen Methoden durchgeführt werden. Beispielsweise wird das Reaktionsgemisch - gegebenenfalls nach Verdünnen mit einem mit Wasser praktisch nicht mischbaren Lösungsmittel, wie z. B. Methylenchlorid - mit verdünnter Salzsäure und mit Wasser gewaschen, getrocknet, filtriert und eingeengt. Das im Rückstand verbliebene Produkt der Formel (I) wird durch Verreiben mit einem geeigneten organischen Lösungsmittel, wie z. B. Ethanol zur Kristallisation gebracht und durch Absaugen isoliert.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria,

Eleocharis, Scirpus, Paspalum, Ischaemum, Spenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben

und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten

Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 4-Amino-6-(1,1-dimethyl-ethyl)-3-methylthio-1,2,4-triazin-5-(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5-(4H)-on, 2-Chlor-4-ethylamino-6-isopropylamino-1,3,5-triazin, das R-Enantiomere des 2-[4-(3,5-Dichlor-pyridyl-2-oxy)-phenoxy]-propionsäure-(trimethylsilyl)-methylesters, das R-Enantiomere des 2-[4-(3,5-Dichlor-pyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxyessigsäure, 2-(2-Methyl-4-chlor-phenoxy)-propionsäure, 3,5-Diiod-4-hydroxy-benzonitril, 3,5-Dibrom-4-hydroxybenzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,005 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

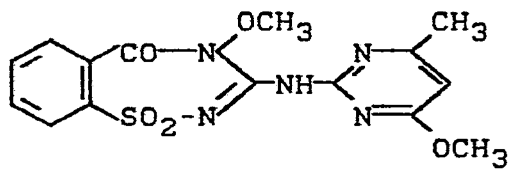

18 g (0,075 Mol) 2-Chlorsulfonyl-benzoylchlorid werden bei -40 °C unter Rühren zu einer Lösung von 16,1 g (0,075 Mol) N'-(4-Methoxy-6-methyl-pyrimidin-2-yl)-N''-methoxy-guanidin und 13 g (0,165 Mol) Pyridin in 75 ml Methylenchlorid getropft. Man rührt dann 2 Stunden bei 20 bis 25 °C nach. Nach dem Waschen der Methylenchloridlösung mit verdünnter Salzsäure, Eiswasser, Trocknen, Filtrieren und Einengen erhält man einen Rückstand, der durch Verreiben mit Methanol zur Kristallisation gebracht wird. Das hierbei angefallene Produkt wird durch Absaugen isoliert.

Man erhält 4,5 g (16 % der Theorie) der Verbindung der oben angegebenen Strukturformel vom Schmelzpunkt 166 °C.

Nach dem im vorausgehenden Beispiel exemplarisch beschriebenen Verfahren können die in der nachstehenden Tabelle 1 aufgeführten Verbindungen der Formel (I) hergestellt werden.

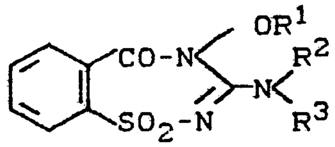

(I)

Tabelle 1

| Beisp.-Nr. | $R^1$ | $R^2$ | $R^3$ | Schmelzpunkt [°C] |
|---|---|---|---|---|
| 2 | $-CH_3$ | H | 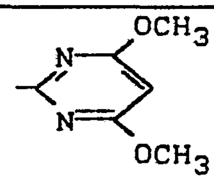 | 180 |
| 3 | $-C_4H_9$-sec. | H | 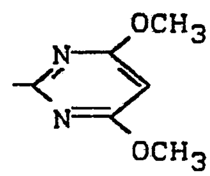 | |
| 4 | $-CH_3$ | H | 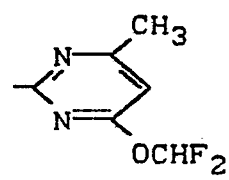 | |
| 5 | $-CH_3$ | H | 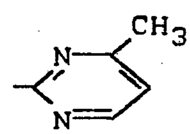 | 172 - 173 |

Tabelle 1-Fortsetzung

| Beisp.-Nr. | $R^1$ | $R^2$ | $R^3$ | Schmelzpunkt [°C] |
|---|---|---|---|---|
| 6 | -$CH_2$- 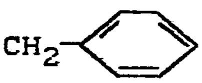 | H | 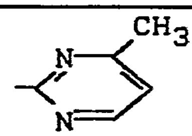 | |
| 7 | $-CH_3$ | H | 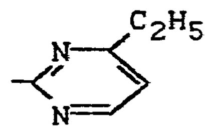 | |
| 8 | $-CH_3$ | H | 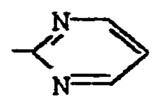 | 110 |
| 9 | $-C_2H_5$ | H | 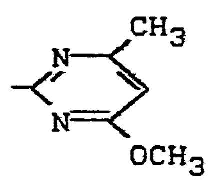 | |
| 10 | $-C_2H_5$ | H | 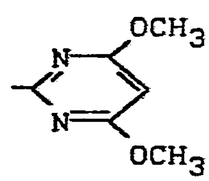 | |
| 11 | -$CH_2$- 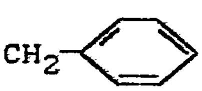 | H | 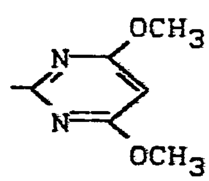 | |

Tabelle 1-Fortsetzung

| Beisp.-Nr. | $R^1$ | $R^2$ | $R^3$ | Schmelzpunkt [°C] |
|---|---|---|---|---|
| 12 | $-CH_2COOC_2H_5$ | H | 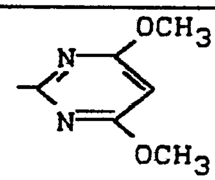 | |
| 13 | $-CH_3$ | H | 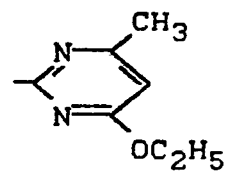 | |
| 14 | $-CH_3$ | H | 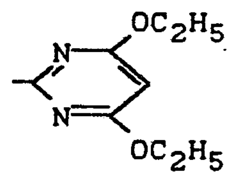 | |
| 15 | $-CH_3$ | H | 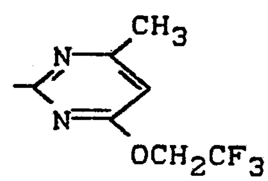 | |
| 16 | $-CH_2-CH=CH_2$ | H | 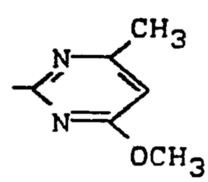 | |

Herstellung von Ausgangsverbindungen der Formel (III)

Beispiel (III-1)

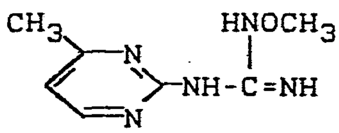

Eine Mischung aus 13,4 g (0,1 Mol) 2-Cyanamino-4-methyl-pyrimidin, 9,2 g (0,11 Mol) O-Methylhydroxylamin-hydrochlorid und 50 ml Ethanol wird 6 Stunden unter Rückfluß erhitzt. Dann wird abgesaugt. Das Filtrat wird eingeengt und der Rückstand mit 200 ml Wasser versetzt. Die wässrige Lösung wird mit Natronlauge auf pH 8 - pH 9 eingestellt. Das hierbei kristallin anfallende Produkt wird durch Absaugen isoliert.

Man erhält 13,1 g (72 % der Theorie) N'-(4-Methyl-pyrimidin-2-yl)-N''-methoxy-guanidin vom Schmelzpunkt 152 °C.

Analog können die in der nachstehenden Tabelle 2 aufgeführten Verbindungen der Formel (III) hergestellt werden:

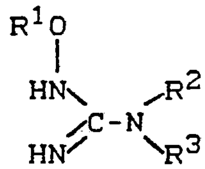

(III)

Tabelle 2

| Beisp.-Nr. | $R^1$ | $R^2$ | $R^3$ | Schmelzpunkt [°C] |
|---|---|---|---|---|
| (III-2) | $-C_2H_5$ | H | 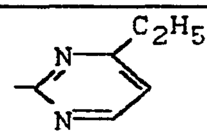 | |
| (III-3) | $-CH_3$ | H | 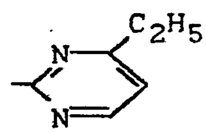 | 98 |
| (III-4) | $-CH_2$- 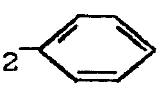 | H | 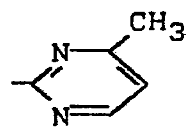 | 150 |
| (III-5) | $-CH_2$- 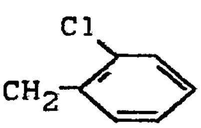 | H | 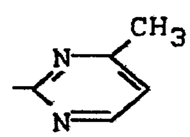 | 140 |

Tabelle 2-Fortsetzung

| Beisp.-Nr. | $R^1$ | $R^2$ | $R^3$ | Schmelzpunkt [°C] |
|---|---|---|---|---|
| (III-6) | $-CH_3$ | H | 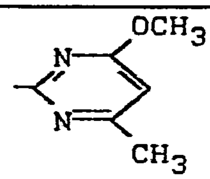 | 126 |
| (III-7) | $-CH_3$ | $-CH_3$ | 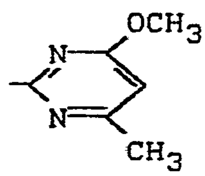 | 135 |
| (III-8) | $-CH_3$ | H | 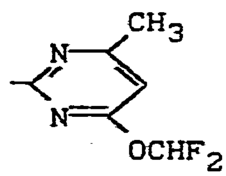 | |
| (III-9) | $-CH_3$ | H | 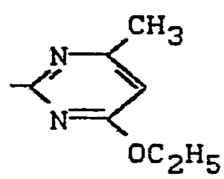 | |
| (III-10) | $-CH_3$ | H | 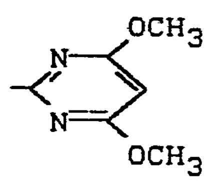 | 122 |
| (III-11) | $-C_4H_9$-sec. | H | 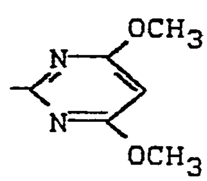 | 68 |

Tabelle 2-Fortsetzung

| Beisp.-Nr. | $R^1$ | $R^2$ | $R^3$ | Schmelzpunkt [°C] |
|---|---|---|---|---|
| (III-12) | $-C_4H_9$-i | H | 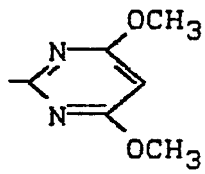 | 76 |
| (III-13) | $-C_2H_5$ | H | 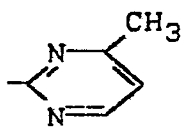 | 95 |
| (III-14) | $-CH_2-COOC_2H_5$ | H | 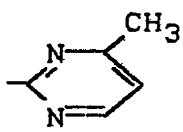 | |
| (III-15) | $-CH_2$- 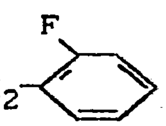 | H | 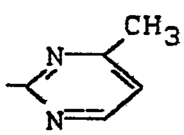 | 205 |
| (III-16) | $-CH_2-CH=CH_2$ | H | 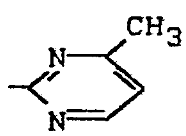 | |
| (III-17) | $-C_4H_9$-n | H | 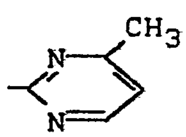 | |

Tabelle 2-Fortsetzung

| Beisp.-Nr. | $R^1$ | $R^2$ | $R^3$ | Schmelzpunkt [°C] |
|---|---|---|---|---|
| (III-18) | $-C_4H_9$-sec. | H | 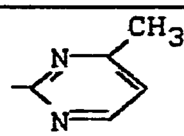 | |
| (III-19) | $-CH_2CH_2CH_2Cl$ | H | 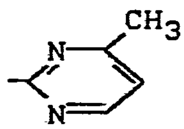 | 102 |
| (III-20) | $-CH_3$ | H | 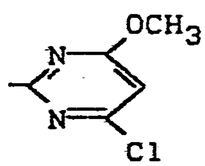 | 112 |
| (III-21) | $-CH_2$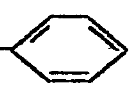 | H | 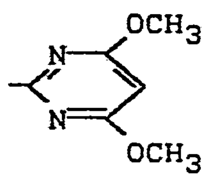 | 74 |
| (III-22) | $-CH_3$ | H | 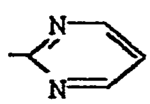 | 107 - 109 |
| (III-23) | $-CH_3$ | H | 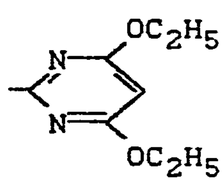 | |

Tabelle 2-Fortsetzung

| Beisp.-Nr. | $R^1$ | $R^2$ | $R^3$ | Schmelzpunkt [°C] |
|---|---|---|---|---|
| (III-24) | $-CH_2$ 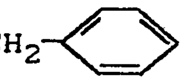 | H | 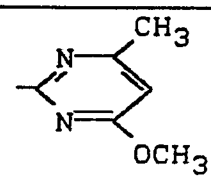 | $n_D^{20}$: 1,5645 |
| (III-25) | $-CH_2$ 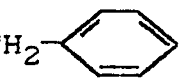 | H | 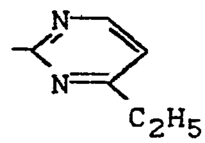 | 112 |
| (III-26) | $-CH$ 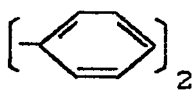 | H | 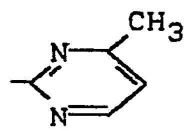 | 165 |
| (III-27) | $-CH_2$ 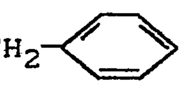 | H | 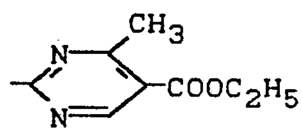 | 130 |
| (III-28) | $-CH_2CH_2OCH_3$ | H | 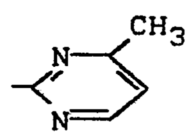 | 242 (Zers.) |
| (III-29) | $-CH_2-CH=CH_2$ | H | 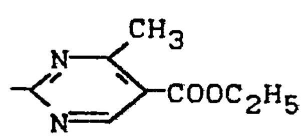 | 143 |
| (III-30) | $-CH_2-CH=CH_2$ | H | 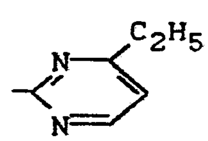 | 83 |

Herstellung von Ausgangsstoffen der Formel (IV)

Beispiel (IV-1)

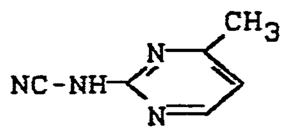

13,2 g (0,1 Mol) Acetylacetaldehyddimethylacetal werden unter Rühren bei 20 - 25 °C zu einer Mischung aus 8,4 g (0,1 Mol) Dicyandiamid, 5,4 g (0,1 Mol) Natriummethylat und 100 ml Methanol getropft. Man erhitzt das Reaktionsgemisch 2 Stunden unter Rückfluß. Nach dem Abddestillieren des Lösungsmittels wird der kristalline Rückstand in 100 ml Wasser gelöst. Es wird filtriert. Das Filtrat wird mit konz. Salzsäure auf pH = 3 eingestellt. Das kristallin ausgefallene Produkt wird durch Absaugen isoliert.

Man erhält 9,7 g (72 % der Theorie) 2-Cyanamino-4-methyl-pyrimidin vom Schmelzpunkt 203 °C.

Beispiel (IV-2)

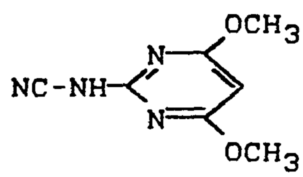

Eine auf 100 °C erhitzte Lösung von 24 g (0,427 Mol) Kaliumhydroxid in 100 ml Wasser wird bei 100 °C unter Rühren zu einer Mischung aus 9,2 g (0,043 Mol) N-(4,6--Dimethoxy-pyrimidin-2-yl)-thioharnstoff und 70 ml Wasser gegeben. Man rührt 2 Minuten bei 100 °C nach und gibt eine auf 100 °C erwärmte Lösung von 16,2 g (0,05 Mol) Blei(II)-acetat in 30 ml Wasser hinzu. Man erhitzt noch 5 Minuten unter Rückfluß, kühlt dann auf 0 °C bis 5 °C ab und versetzt die wässrige Lösung mit 30 ml Eisessig. Das hierbei kristallin ausfallende Produkt wird durch Absaugen isoliert.

Man erhält 6,3 g (81,5 % der Theorie) 2-Cyanamino-4,6--dimethoxy-pyrimidin vom Schmelzpunkt 202 °C.

Zum gleichen Ergebnis kommt man, wenn man in Wasser- Methanol-Gemischen unter sonst gleichen Bedingungen arbeitet.

Nach den in den vorausgehenden Beispielen exemplarisch beschriebenen Verfahren können die in der nachstehenden Tabelle 3 aufgeführten Verbindungen der Formel (IV) hergestellt werden:

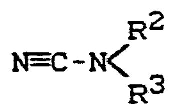 (IV)

Tabelle 3

| Beisp.-Nr. | $R^2$ | $R^3$ | Schmelzpunkt [°C] |
|---|---|---|---|
| (IV-3) | H | 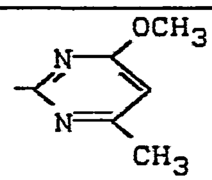 | 258 |
| (IV-4) | H | 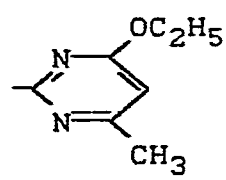 | |
| (IV-5) | H | 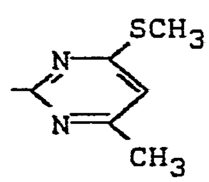 | |
| (IV-6) | H | 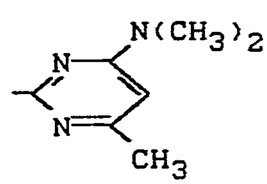 | |

Tabelle 3-Fortsetzung

| Beisp.-Nr. | $R^2$ | $R^3$ | Schmelzpunkt [°C] |
|---|---|---|---|
| (IV-7) | H | 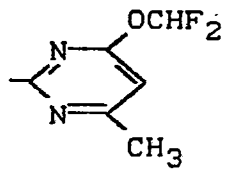 | 174 |
| (IV-8) | H | 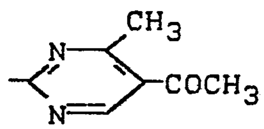 | 174 |
| (IV-9) | H | 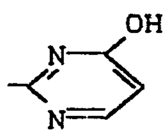 | > 300 |
| (IV-10) | H | 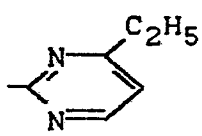 | 146 |
| (IV-11) | H | 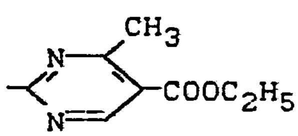 | 126 |
| (IV-12) | H | 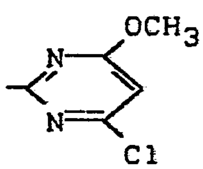 | 200 |

Tabelle 3-Fortsetzung

| Beisp.-Nr. | $R^2$ | $R^3$ | Schmelzpunkt [°C] |
|---|---|---|---|
| (IV-13) | H | 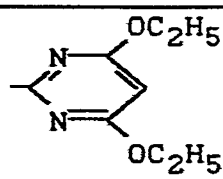 | 235 - 237 |
| (IV-14) | H | 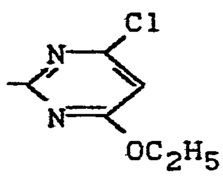 | |
| (IV-15) | H | 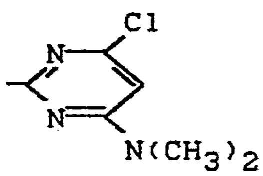 | |
| (IV-16) | H | 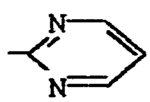 | 186 |
| (IV-17) | H | 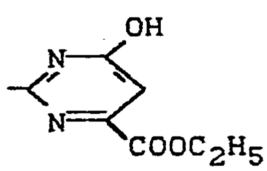 | 220 (Zers.) |

2-(Alkyl-cyano-amino)-pyrimidine der Formel (IV) können beispielsweise wie folgt hergestellt werden:

Beispiel (IV-18)

----------------

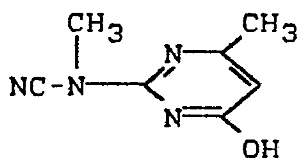

12,6 g (0,1 Mol) Dimethylsulfat werden zu einer Lösung von 15 g (0,1 Mol) 2-Cyanamino-4-hydroxy-6-methyl-pyrimidin - hergestellt nach Verfahren (b) - und 4,1 g (0,1 Mol) Natriumhydroxid in 60 ml Wasser tropfenweise gegeben, wobei die Reaktionstemperatur von 20 °C auf 40 °C steigt. Nach zweistündigem Rühren bei 20 °C wird das kristallin angefallene Produkt durch Absaugen isoliert.

Man erhält 11,1 g (68 % der Theorie) 2-(Methyl-cyano--amino)-4-hydroxy-6-methyl-pyrimidin vom Schmelzpunkt 290 °C.

Analog erhält man:

Beispiel (IV-19)

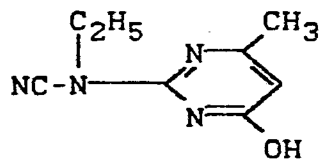

Fp. 215 °C bis 220 °C

Beispiel (IV-20)

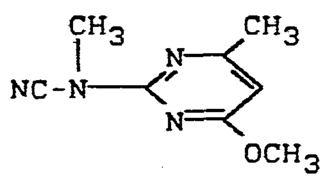

127,5 g (1 Mol) Dimethylsulfat werden zu einer Lösung von 75 g (0,5 Mol) 2-Cyanamino-4-hydroxy-6-methyl-pyrimidin - hergestellt nach Verfahren (b) - und 44 g (1,1 Mol) Natriumhydroxid in 750 ml Wasser tropfenweise gegeben, wobei die Reaktionstemperatur von 20 °C auf 35 °C ansteigt. Nach zwölfstündigem Rühren bei 20 °C wird durch Zugabe von Natronlauge ein pH-Wert zwischen 9 und 10 eingestellt und das kristallin angefallene Produkt durch Absaugen isoliert.

Man erhält 13 g (15 % der Theorie) 2-(Methyl-cyano-amino)-4-methoxy-6-methyl-pyrimidin vom Schmelzpunkt 123 °C.

Analog erhält man:

Beispiel (IV-21)

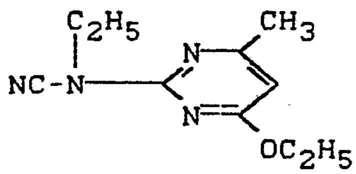

Fp. 71 °C

Herstellung der Ausgangsstoffe der Formel (X)

Beispiel (X-1)

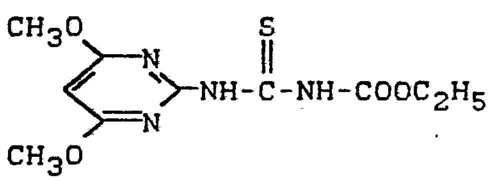

Eine Mischung aus 15,5 g (0,1 Mol) 2-Amino-4,6-dimethoxy-pyrimidin, 13,1 g (0,1 Mol) Ethoxycarbonyl-isothiocyanat und 200 ml Acetonitril wird 2 Stunden bei 60 °C gerührt. Dann wird auf 10 °C abgekühlt, und das kristallin angefallene Produkt wird durch Absaugen isoliert.

Man erhält 22,5 g (79 % der Theorie) 1-(Ethoxycarbonyl)-3-(4,6-dimethoxy-pyrimidin-2-yl)-thioharnstoff vom Schmelzpunkt 194 °C (Zers.).

Nach dem im vorausgehenden Beispiel exemplarisch beschriebenen Verfahren können die in der nachstehenden Tabelle 4 aufgeführten Verbindungen der Formel (X) hergestellt werden:

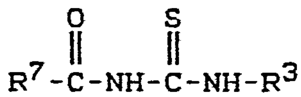 (X)

Tabelle 4

| Beisp.-Nr. | $R^7$ | $R^3$ | Schmelzpunkt [°C] |
|---|---|---|---|
| (X-2) | 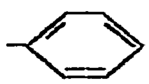 | 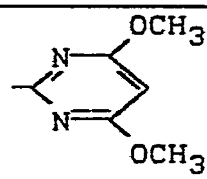 | 189 |
| (X-3) | 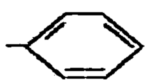 | 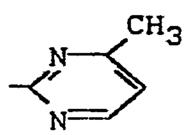 | 198 - 199 (Zers.) |
| (X-4) | $-OC_2H_5$ | 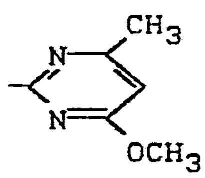 | 217 |
| (X-5) | 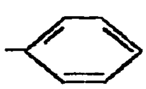 | 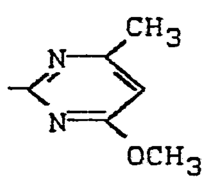 | 190 |

Tabelle 4-Fortsetzung

| Beisp.-Nr. | $R^7$ | $R^3$ | Schmelzpunkt [°C] |
|---|---|---|---|
| (X-6) | $-OC_2H_5$ | 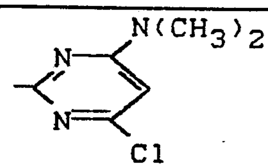 | 168 |
| (X-7) | 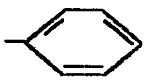 | 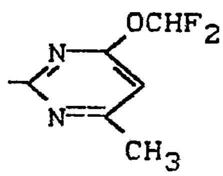 | 182 |
| (X-8) | $-OC_2H_5$ | 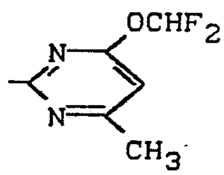 | 184 - 185 |
| (X-9) | $-OC_2H_5$ | 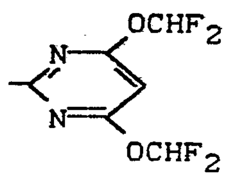 | 173 |
| (X-10) | $-OC_2H_5$ | 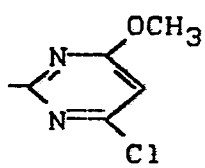 | 160 - 162 |
| (X-11) | $-OC_2H_5$ | 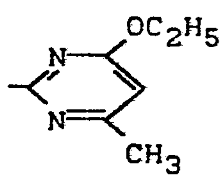 | |

Tabelle 4-Fortsetzung

| Beisp.-Nr. | $R^7$ | $R^3$ | Schmelzpunkt [°C] |
|---|---|---|---|
| (X-12) | $-OC_2H_5$ | 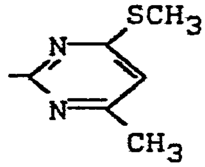 | |
| (X-13) | $-OC_2H_5$ | 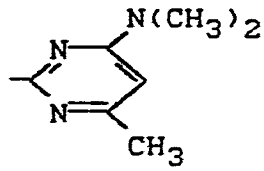 | |
| (X-14) | 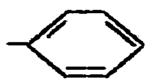 | 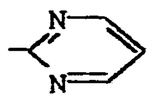 | 173 |
| (X-15) | 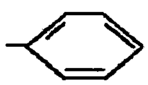 | 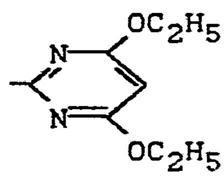 | 179 |
| (X-16) | $-OC_2H_5$ | 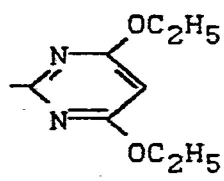 | 159 |
| (X-17) | 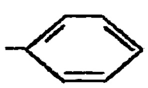 | 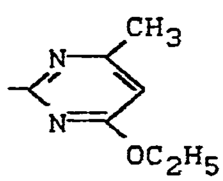 | 156 |

Tabelle 4-Fortsetzung

| Beisp.-Nr. | $R^7$ | $R^3$ | Schmelzpunkt [°C] |
| --- | --- | --- | --- |
| (X-18) | 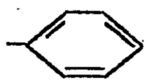 | 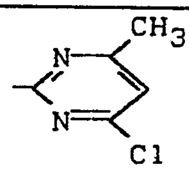 | 144 |
| (X-19) | $-OC_2H_5$ | 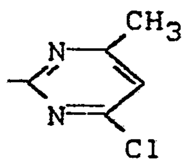 | 122 - 124 |

## Herstellung der Ausgangsstoffe der Formel (XI)

Beispiel (XI-1)

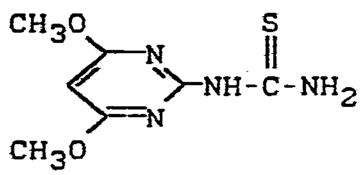

Eine Mischung aus 5,0 g (0,0175 Mol) 1-(Ethoxycarbonyl)-3-(4,6-dimethoxy-pyrimidin-2-yl)-thioharnstoff, 4,0 g (0,1 Mol) Natriumhydroxid und 100 ml Wasser wird 2 Tage bei 20 °C gerührt. Dann wird unter Rühren solange verdünnte Salzsäure zugetropft, bis die Lösung sauer gestellt ist und die $CO_2$-Entwicklung beendet ist. Das kristallin anfallende Produkt wird durch Absaugen isoliert.

Man erhält 3,5 g (94 % der Theorie) 4,6-Dimethoxy-pyrimidin-2-yl-thioharnstoff vom Schmelzpunkt 245 - 248 °C (Zers.).

Nach dem im vorausgehenden Beispiel exemplarisch beschriebenen Verfahren können die in der nachstehenden Tabelle 5 aufgeführten Verbindungen der Formel (XI) hergestellt werden:

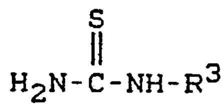 (XI)

Tabelle 5

| Beispiel Nr. | $R^3$ | Schmelzpunkt [°C] |
|---|---|---|
| (XI-2) | 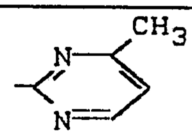 | 264 - 265 |
| (XI-3) | 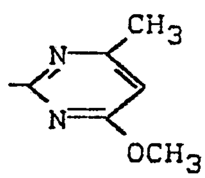 | 207 |
| (XI-4) | 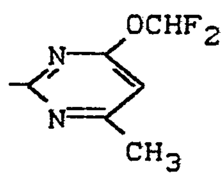 | 192 - 194 |
| (XI-5) | 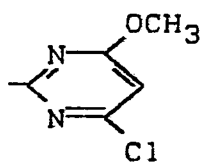 | 225 - 227 (Zers.) |

Tabelle 5-Fortsetzung

| Beispiel Nr. | $R^3$ | Schmelzpunkt [°C] |
|---|---|---|
| (XI-6) | 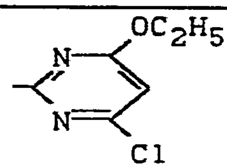 | |
| (XI-7) | 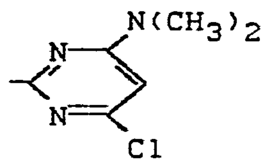 | |
| (XI-8) | 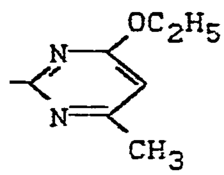 | |
| (XI-9) | 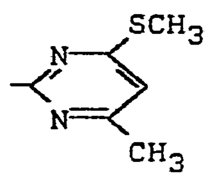 | |
| (XI-10) | 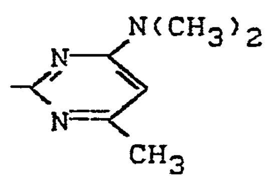 | |
| (XI-11) | 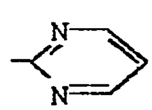 | 263 |

Tabelle 5-Fortsetzung

| Beispiel Nr. | $R^3$ | Schmelzpunkt [°C] |
|---|---|---|
| (XI-12) | 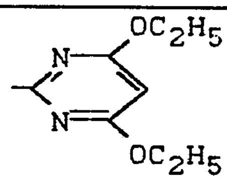 | 166 |
| (XI-13) | 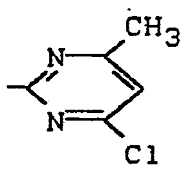 | > 180 (Zers.) |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit.

## Patentansprüche

1) Benzolactamsultame der allgemeinen Formel (I)

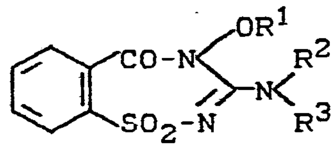

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für den Rest 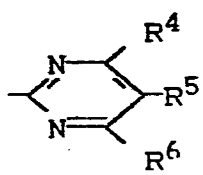 steht, worin

$R^4$ für Wasserstoff, Hydroxy, Halogen oder einen gegebenenfalls substituierten Rest aus der Reihe Alkyl, Alkoxy und Alkylthio steht,

$R^5$ für Wasserstoff, Halogen, Cyano, Formyl oder einen gegebenenfalls substituierten Rest aus der Reihe Alkyl, Alkylcarbonyl und Alkoxycarbonyl steht und

$R^6$ für Wasserstoff, Amino oder einen gegebenenfalls substituierten Rest aus der Reihe Alkyl, Alkoxy, Alkylamino und Dialkylamino steht,

- mit der Maßgabe, daß $R^4$ und $R^6$ nicht gleichzeitig für Methyl stehen.

2) Verfahren zur Herstellung von Benzolactamsultamen der allgemeinen Formel (I) gemäß Anspruch 1, dadurch gekennzeichnet, daß man 2-Chlorsulfonyl-benzoylchlorid der Formel (II)

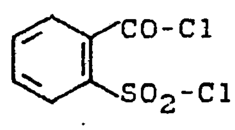

(II)

mit Oxyguanidin-Derivaten der Formel (III)

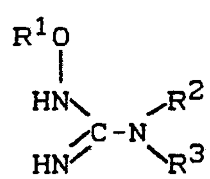

(III)

in welcher

$R^1$, $R^2$ und $R^3$ die oben angegebenen Bedeutungen haben,

in Gegenwart von Säureakzeptoren und gegebenenfalls in Gegenwart von Verdünnungsmitteln umsetzt.

3) Herbizide Mittel, gekennzeichnet durch einen Gehalt an mindestens einem Benzolactamsultam der allgemeinen Formel (I) gemäß Anspruch 1.

4) Verwendung von Benzolactamsultamen der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man Benzolactamsultame der allgemeinen Formel (I) gemäß Anspruch 1 mit Streckmitteln und/oder oberflächenaktiven Mitteln vermischt.

0203220

Europäisches Patentamt

# EUROPÄISCHER RECHERCHENBERICHT

Nummer der Anmeldung

EP 85 11 0829

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 007 687 (DU PONT DE NEMOURS)<br>* Seiten 198-214, Patentansprüche *<br>--- | 1-5 | C 07 D 417/12<br>A 01 N 43/72 |
| A | FR-A-1 571 016 (UPJOHN)<br>* Das ganze Dokument *<br>--- | 1-5 | |
| D,E | EP-A-0 173 958 (BAYER)<br>* Seiten 33-37 *<br>----- | 1,2 | |
| | | | **RECHERCHIERTE SACHGEBIETE (Int. Cl.4)** |
| | | | C 07 D 417/00<br>C 07 D 285/00 |

Der vorliegende Recherchenbericht wurde für alle Patentansprüche erstellt.

| Recherchenort | Abschlußdatum der Recherche | Prüfer |
|---|---|---|
| DEN HAAG | 04-09-1986 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument